# EUROPÄISCHE PATENTANMELDUNG

(11) **EP 0 717 044 A1**
(43) Veröffentlichungstag der Anmeldung: **19.06.1996**
(21) Anmeldenummer: 95119009.9
(22) Anmeldetag: 02.12.1995
(51) Int. Cl.: C07D 495/04, A61K 31/44

(54) **Thienopyridonderivate, deren Herstellung und deren Verwendung als NMDA-Rezeptor Antagonisten**

(30) Priorität: 15.12.1994 DE 4444815
(71) Anmelder: MERCK PATENT GmbH, D-64293 Darmstadt (DE)
(72) Erfinder: Gottschlich, Rudolf, Dr., D-64354 Reinheim (DE); Leibrock, Joachim, Dr., D-64347 Griesheim (DE); Noe, Christian, Dr., D-60596 Frankfurt (DE); Berger, Michael, Dr., A-1180 Wien (DE); Buchstaller, Hans-Peter, D-65843 Sulzbach/Ts (DE)

(57) **Zusammenfassung**

Thienopyridonderivate der Formel I 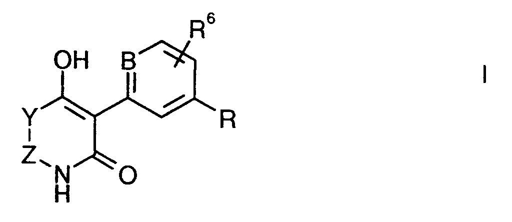
worin
- B: CH oder N, 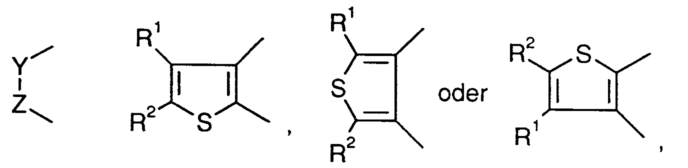
- R: H, 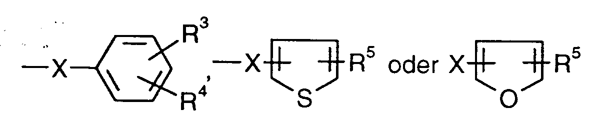
- R¹, R² und R⁵: jeweils H, A oder Hal,
- R³ und R⁴: jeweils H, A, OH, OA, Hal, CF₃, NO₂, NH₂, NHA, N(A)₂ oder NHAc,
- R⁶: H oder Hal,
- X: -CH₂-, -CO-, -O-, -NH-, -NA- oder S,
- A: Alkyl mit 1-4 C-Atomen,
- Ac: Alkanoyl mit 1-6 C-Atomen oder Benzoyl und
- Hal: F, Cl, Br oder I bedeuten,
sowie deren Salze zeigen eine hohe Affinität zu Bindungsstellen von Aminosäure-Rezeptoren und eignen sich daher zur Behandlung von neurodegenerativen Erkrankungen.

## Beschreibung

Die Erfindung betrifft neue Thienopyridonderivate der Formel I 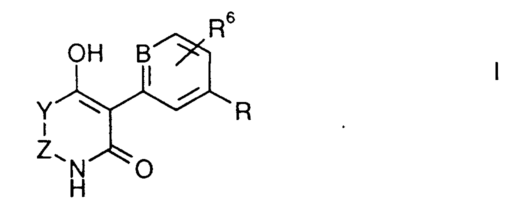
worin
- B: CH oder N, 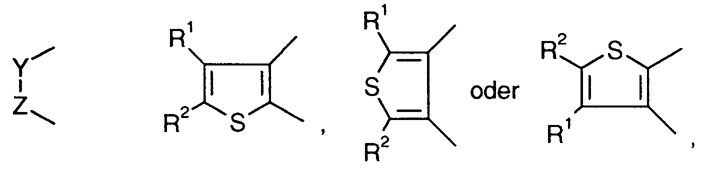
- R: H, 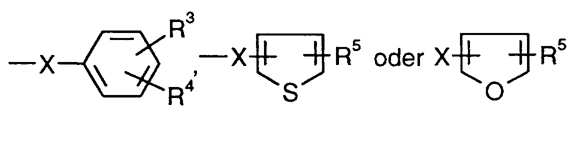
- R¹, R² und R⁵: jeweils H, A oder Hal,
- R³ und R⁴: jeweils H, A, OH, OA, Hal, CF₃, NO₂, NH₂, NHA, N(A)₂ oder NHAc,
- R⁶: H oder Hal,
- X: -CH₂-, -CO-, -O-, -NH-, -NA- oder S,
- A: Alkyl mit 1-4 C-Atomen,
- Ac: Alkanoyl mit 1-6 C-Atomen oder Benzoyl und
- Hal: F, Cl, Br oder I
bedeuten,
sowie ihre Salze.

Ähnliche Verbindungen sind aus Journal of Medicinal Chemistry 1994, 37, 1402-1405, bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze wertvolle pharmakologische Eigenschaften besitzen. Sie zeigen eine hohe Affinität zu Bindungsstellen von Aminosäure-Rezeptoren, insbesondere zur Glycin-, Polyamin- und/oder NMDA-Bindungsstelle des NMDA-Rezeptors (NMDA = N-Methyl-D-aspartat). Die Verbindungen eignen sich daher zur Behandlung von neurodegenerativen Erkrankungen einschließlich cerebrovaskularen Krankheiten. Ebenso können die neuen Wirkstoffe als Analgetika oder Anxiolytika sowie zur Behandlung von Epilepsie, Schizophrenie, der Alzheimer-, der Parkinson- bzw. Huntington-Krankheit, cerebralen Ischämien oder Infarkten verwendet werden. Ferner eignen sie sich zur Behandlung von Psychosen, bedingt durch überhöhte Aminosäurespiegel.

Der [³H]-CGP-39653-Bindungstest für die Glutamat-Bindungsstelle des NMDA-Rezeptors kann beispielsweise nach der Methode von M.A. Stils et al., beschrieben in Eur. J. Pharmacol. 192, 19-24 (1991), durchgeführt werden. Der Test für die Glycin-Bindungsstelle des NMDA-Rezeptors ist nachweisbar durch die Methode von M.B. Baron et al., beschrieben in Eur. J. Pharmacol. 206, 149-154 (1991). Die in-vitro-Aminosäurefreisetzung ist gemäß der Methode von D. Lobner und P. Lipton (Neurosci. Lett. 117, 169-174 (1990)) nachweisbar.

Die Wirkung gegen Morbus Parkinson, d.h. die Potenzierung des L-DOPA-induzierten kontralateralen Drehens bei hemiparkinsonischen Ratten, ist nach der Methode von U. Ungerstedt und G.W. Arbuthnott, Brain Res 24, 485 (1970) nachweisbar.

Besonders geeignet sind die Verbindungen zur Behandlung oder Prophylaxe von Schlaganfällen sowie zum Schutz vor und zur Behandlung von Hirnödemen und Unterversorgungszuständen des Zentralnervensystems, vor allem Hypoxie oder Anoxie.

Die genannten Wirkungen können außerdem nach den Methoden nachgewiesen oder überprüft werden, wie sie in den folgenden Literaturstellen beschrieben sind:

J.W. McDonald, F.S. Silverstein und M.v. Johnston, Eur. J. Pharmacol. 140, 359 (1987); R. Gill, A.C. Foster und G.N. Woodruff, J. Neurosci. 7, 3343 (1987); S.M. Rothmann, J.H. Thurston, R.E. Hauhart, G.D. Clark und J.S. Soloman, Neurosci. 21, 73 (1987) oder M.P. Goldbert, P.-C. Pham und D.W. Choi, Neurosci. Lett. 80, 11 (1987).

Die Verbindungen können daher als Arzneimittelwirkstoffe in der Human- und Veterinärmedizin verwendet werden. Ferner eignen sie sich als Zwischenprodukte zur Herstellung anderer Verbindungen mit wertvollen Eigenschaften.

Gegenstand der Erfindung sind die Verbindungen der Formel I und ihre Salze sowie ein Verfahren zur Herstellung dieser Verbindungen sowie ihrer Salze, dadurch gekennzeichnet,daß man

### (a) eine Verbindung der Formel II

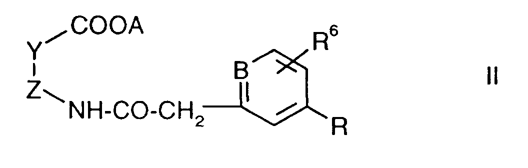

worin
A, B, R, R⁶ und -Y-Z die oben angegebenen Bedeutungen haben,
mit einem cyclisierenden Mittel behandelt,
und daß man gegebenenfalls in einer Verbindung der Formel I einen Rest R in einen anderen Rest R umwandelt und/oder eine Verbindung der Formel I durch Behandeln mit einer Säure oder Base in eines ihrer Salze überführt.

Vor- und nachstehend haben die Reste bzw. Parameter B, R, R¹ bis R⁵, X, -Y-Z-, A, Ac und Hal die bei der Formel I angegebenen Bedeutungen, falls nicht ausdrücklich etwas anderes angegeben ist.

In den vorstehenden Formeln bedeutet A bevorzugt Methyl, Ethyl, Propyl, Isopropyl, weiterhin Butyl, Isobutyl, sek.-Butyl oder tert.Butyl.

Ac bedeutet bevorzugt Formyl, Acetyl, Propionyl oder Benzoyl, ferner z.B. Butyryl, Isobutyryl, Pentanoyl oder Hexanoyl.

Hal bedeutet vorzugsweise Fluor oder Chlor, ferner auch Brom oder Iod.

Der Rest X bedeutet vorzugsweise -CH₂-, -CO-, -O-, -NH-, weiterhin -NA- oder -S-.

B bedeutet vorzugsweise CH, aber auch N.

Die Gruppe -Y-Z- bedeutet vorzugsweise 4-R¹-5-R²-thiophen-2,3-diyl, weiterhin bevorzugt 2-R¹-5-R²-thiophen-3,4-diyl oder 3-R¹-2-R²-thiophen-4,5-diyl.

R ist vorzugsweise H, weiterhin bevorzugt unsubstituiertes oder monosubstituiertes Phenoxy, im einzelnen bevorzugt o-, m- oder p-Methylphenoxy, o-, m- oder p-Methoxyphenoxy, o-, m- oder p-Fluorphenoxy, o-, m- oder p-Chlorphenoxy, ferner bevorzugt o-, m- oder p-Trifluormethylphenoxy.

R ist weiterhin bevorzugt unsubstituiertes oder monosubstituiertes Benzyl, im einzelnen bevorzugt o-, m- oder p-Methylbenzyl, o-, m- oder p-Methoxybenzyl, o-, m- oder p-Fluorbenzyl, o-, m- oder p-Chlorbenzyl, ferner bevorzugt o-, m- oder p-Trifluormethylbenzyl. R ist weiterhin bevorzugt unsubstituiertes oder monosubstituiertes Benzoyl, im einzelnen bevorzugt o-, m- oder p-Methylbenzoyl, o-, m- oder p-Methoxybenzoyl, o-, m- oder p-Fluorbenzoyl, o-, m- oder p-Chlorbenzoyl, ferner bevorzugt o-, m- oder p-Trifluormethylbenzoyl. R bedeutet weiterhin bevorzugt unsubstituiertes oder monosubstituiertes Anilino, im einzelnen bevorzugt o-, m- oder p-Methylanilino, o-, m- oder p-Methoxyanilino, o-, m- oder p-Fluoranilino, o-, m- oder p-Chloranilino, ferner bevorzugt o-, m- oder p-Trifluormethylanilino.

R ist darüber hinaus bevorzugt, o-, m- oder p-Nitrophenoxy, o-, m- oder p-N,N-Dimethylaminophenoxy, o-, m- oder p-Acetamidophenoxy, o-, m- oder p-Nitrobenzyl, o-, m- oder p-N,N-Dimethylaminobenzyl, o-, m- oder p-Acetamidobenzyl, o-, m- oder p-Nitrobenzoyl, o-, m- oder p-N,N-Dimethylaminobenzoyl, o-, m- oder p-Acetamidobenzoyl, o-, m- oder p-Nitroanilino, o-, m- oder p-N,N-Dimethylaminoanilino, o-, m- oder p-Acetamidoanilino.

R¹, R² und R⁵ sind vorzugsweise H, Methyl, Ethyl, Chlor, ferner Propyl oder Brom, R³ und R⁴ sind jeweils vorzugsweise H, ferner Methyl, Methoxy, Fluor, Chlor oder Trifluormethyl.

R⁶ bedeutet vorzugsweise H oder F, ferner auch Br.

Die Verbindungen der Formel I können eine oder mehrere chirale Zentren besitzen und daher in verschiedenen - optisch-aktiven oder optisch-inaktiven - Formen vorkommen. Die Formel I umschließt alle diese Formen.

Einige bevorzugte Gruppen von Verbindungen können durch die folgenden Teilformeln Ia bis Ie ausgedrückt werden, die der Formel I entsprechen und worin die nicht näher bezeichneten Reste die bei Formel I angegebenen Bedeutungen haben, worin jedoch:
in Ia
   - B: CH bedeutet;
in Ib
   - B: N bedeutet;
in Ic
   - -Y-Z-: 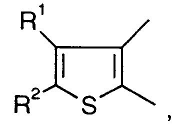
   - R¹: H oder CH₃,
   - R²: H, CH₃, C₂H₅, Cl oder Br,
   - R³: H, CH₃, OH, OCH₃, F, Cl, CF₃, NO₂, NH₂, N(CH₃)₂ oder NHCOCH₃,
   - R⁴: H,
   - R⁵: H und
   - X: -CH₂-, -CO-, -O- oder -NH-
   bedeuten;
in Id
   - -Y-Z-: 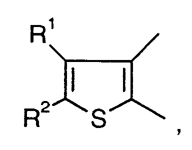
   - R: H oder 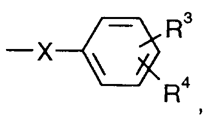
   - R¹: H oder CH₃,
   - R²: H, CH₃, C₂H₅, Cl oder Br,
   - R³: H, CH₃, OCH₃, F, Cl oder CF₃,
   - R⁴: H und
   - X: -CH₂-, -CO-, -O- oder -NH-
   bedeuten;
in Ie
   - B: CH,
   - -Y-Z-: 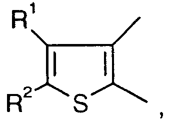
   - R: H,
   - R¹: H oder CH₃ und
   - R²: H, CH₃, C₂H₅, Cl oder Br
   bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in Journal of Medicinal Chemistry 1994, 37, 1402-1405 beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können durch Behandlung einer Verbindung der Formel II mit einem Cyclisierungsmittel, vorzugsweise einer Base erhalten werden. Als Base dienen z.B. ein Kalium- oder Natriumalkoholat wie Kalium- oder Natriummethylat, -ethylat oder -tert.-butylat in einem inerten Lösungsmittel, vorzugsweise dem zugrundeliegenden Alkohol. NaH in Dimethylformamid (DMF) oder KN[Si(CH₃)₃]₂ in einem inerten Lösungsmittel.

Die Cyclisierung erfolgt zweckmäßig bei Temperaturen zwischen etwa -100 und etwa +160°; vorzugsweise arbeitet man zwischen -85 und +50°.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Carbonsäuren wie Ameisensäure oder Essigsäure; Ester wie Ethylacetat; Amide wie DMF, Dimethylacetamid oder Phosphorsäurehexamethyltriamid (HMPT); Sulfoxide wie Dimethylsulfoxid; Schwefelkohlenstoff; chlorierte Kohlenwasserstoffe wie Methylenchlorid, Chloroform, Trichlorethylen, 1,2-Dichlorethan oder Kohlenstofftetrachlorid; Kohlenwasserstoffe wie Hexan, Petrolether, Benzol Toluol oder Xylol. Weiterhin eignen sich Gemische dieser Lösungsmittel untereinander.

In den Verbindungen der Formel II bedeutet A vorzugsweise Methyl, ferner Ethyl.

Die Ausgangsverbindungen der Formel II sind in der Regel neu. Sie können aber nach an sich bekannten Methoden hergestellt werden. So kann z.B. 2-Amino-4-methyl-thiophen-3-carbonsäuremethylester mit z.B. Phenylessigsäurechlorid in einem inerten Lösungsmittel umgesetzt werden, wobei 2-Phenylacetamido-4-methyl-thiophen-3-carbonsäuremethylester entsteht. Dies erfolgt zweckmäßig bei Temperaturen zwischen etwa 0 und etwa 200°; vorzugsweise arbeitet man zwischen 60 und 90°.

Es ist ferner möglich, eine Verbindung der Formel I in eine andere Verbindung der Formel I umzuwandeln, indem man den Rest R in einen anderen Rest umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt.

So kann man beispielsweise freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°. Analog können freie Hydroxygruppen alkyliert werden.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z.B. eine Verbindung der Formel I, die eine NHCOA- oder eine OA-Gruppe enthält in die entsprechende Verbindung der Formel I umgewandelt werden, die stattdessen eine NH₂- oder eine OH-Gruppe enthält.

Acylierte Amine der Formel I, in denen der Phenyl- oder Pyridinrest einfach durch NHCOAlkyl substituiert sind, können gespalten werden, wobei die entsprechenden Aminoderivate entstehen. Z.B. kann man die Acylaminoverbindungen spalten durch Behandeln mit methanolischer Kalilauge bei etwa 20-140°.

Ether der Formel I, in denen der Phenyl- oder Pyridylrest einfach durch O-Alkyl substituiert ist, können gespalten werden, wobei die entsprechenden Hydroxyderivate entsehen. Z.B. kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250°, oder durch Behandeln mit Diisobutylaluminiumhydrid in Toluol bei etwa 0-110°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilsierungs- und/ oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Schmerzzuständen, aber auch zur Minderung der Folgeschäden nach einer Ischämie verwendet werden. Besonders eignen sich die Verbindungen zur Behandlung von neurodegenerativen Erkrankungen bzw. von Erkrankungen, die durch eine Fehlfunktion an der Glycin-, Polyamin- oder Glutamat-Bindungsstelle des NMDA-Rezeptors hervorgerufen werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. Rf-Werte an Kieselgel.

### Beispiel 1

Eine Lösung von 0,6 g 5-Chlor-4-methyl-2-(phenylacetamido)-thiophen-3-carbonsäuremethylester (F. 105-107°; erhältlich durch Chlorierung der entsprechenden chlorfreien Verbindung mit Hilfe von N-Chlorsuccinimid) in 20 ml THF wird auf -70° gekühlt. Anschließend werden 8,16 ml einer 0,5 molaren Lösung von KN(Si(CH₃)₃)₂ in Toluol zugetropft. Die Reaktionslösung wird langsam auf Raumtemperatur gebracht und dann engedampft. Der Rückstand wird in Wasser aufgenommen und mehrmals mit Diethylether extrahiert. Die wäßrige Phase wird dann mit 2 N HCl angesäuert und der Niederschlag abfiltriert, mit Wasser gewaschen und nach Digerieren mit Diethylether erneut abfiltriert und getrocknet. Man erhält 2-Chlor-3-methyl-4-hydroxy-5-phenyl-6,7-dihydrothieno-[2,3-b]pyridin-6-on, Rf (CH₂Cl₂/MeOH 10:1) 0,43.

### Beispiele 2 bis 8

Analog Beispiel 1 erhält man aus den nachstehenden 2-Phenylacetamido-4-R¹-5-R²-thiophen-3-carbonsäure-methyl- bzw. ethylestern:

| Bsp. | R¹ | R² | A | F. |
|---|---|---|---|---|
| 2 | Me | H | Me | 105,5-106,5° |
| 3 | H | Me | Me | 110,5-111,5° |
| 4 | Me | Me | Et | 84-86° |
| 5 | H | Et | Me | 105-106° |
| 6 | H | H | Me | 90-92° (bekannt!) |
| 7 | Me | Br | Me | 105-106,5° |
| 8 | H | Br | Me | - |

die nachstehenden 2-R²-3-R¹-4-hydroxy-5-phenyl-6,7-dihydrothieno[2,3-b]pyridin-6-one:

| Bsp. | R¹ | R² | Rf (CH₂Cl₂/MeOH 10:1) |
|---|---|---|---|
| 2 | Me | H | 0,43 |
| 3 | H | Me | 0,36 |
| 4 | Me | Me | 0,49 |
| 5 | H | Et | 0,44 |
| 6 | H | H | 0,31 |
| 7 | Me | Br | 0,38 |
| 8 | H | Br | 0,34 |

### Beispiele 9 bis 72

Analog Beispiel 1 erhält man aus den nachstehenden 2-(3-R-Phenyl)-acetamido-4-methyl-5-chlor-thiophen-3-carbonsäure-methylestern:

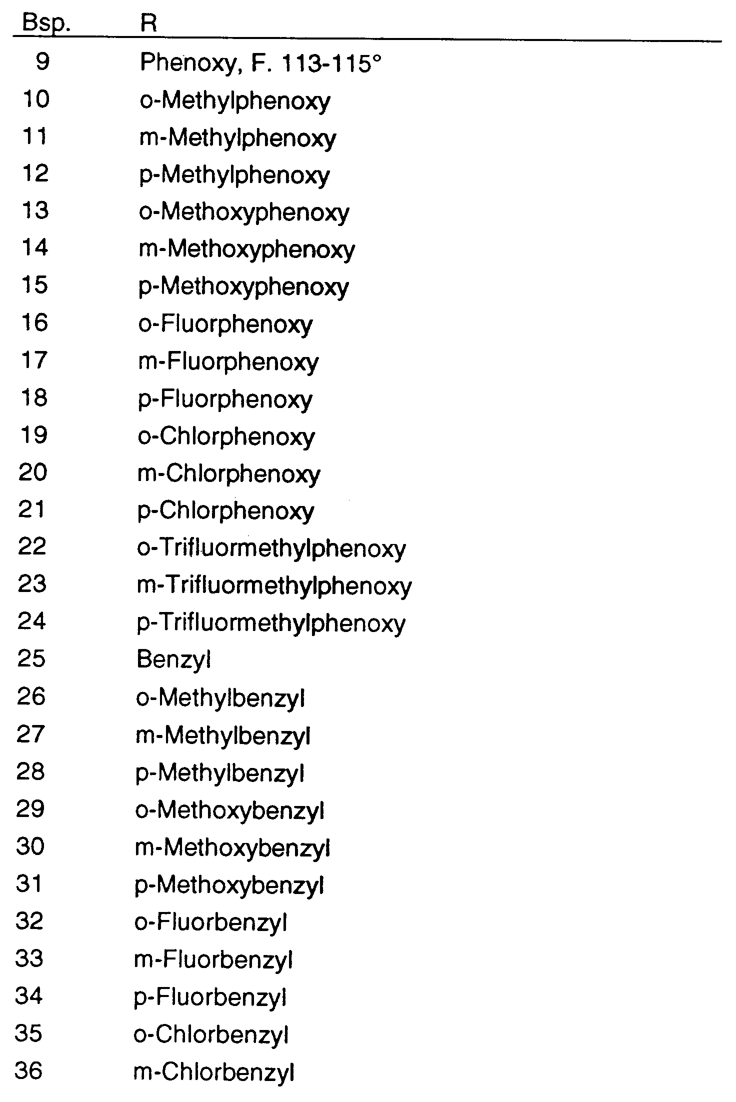

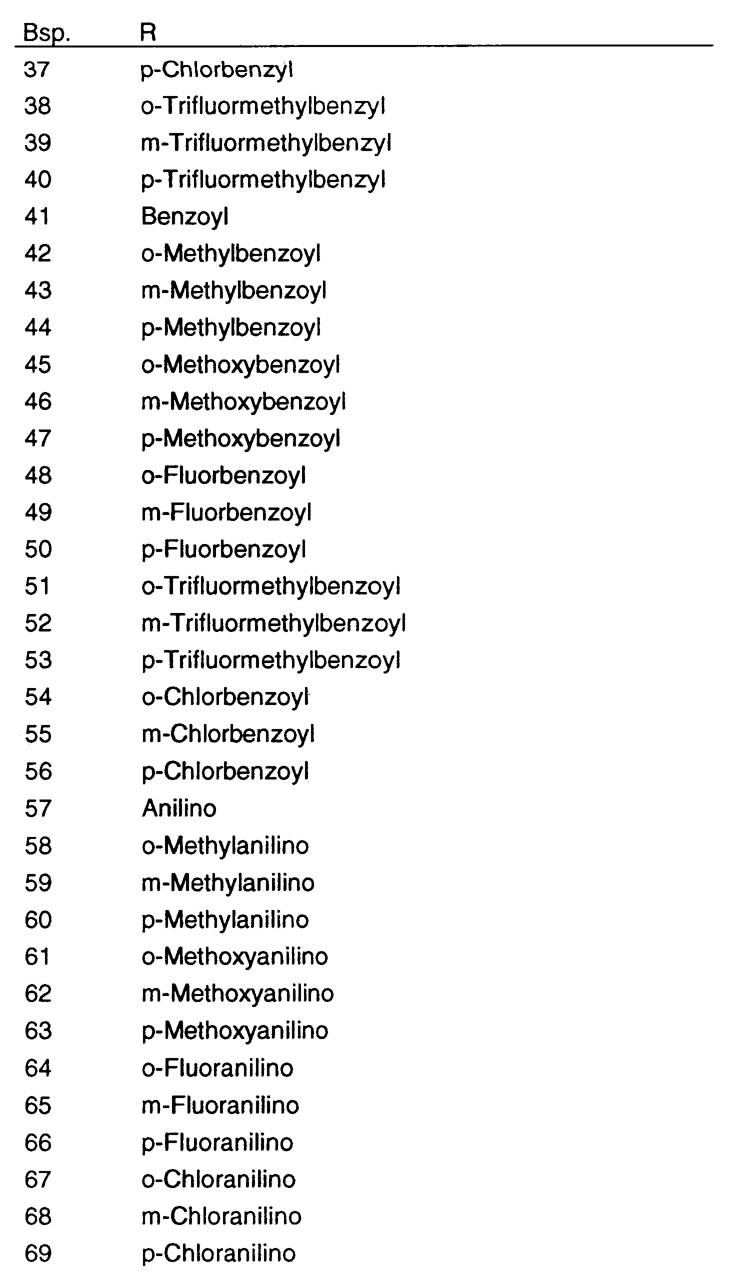

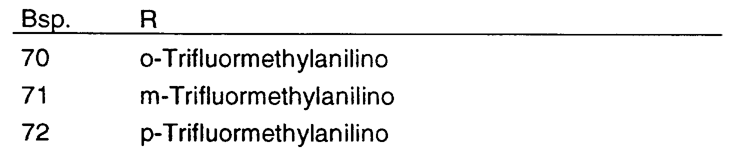

die nachstehenden 2-Chlor-3-methyl-4-hydroxy-5-(3-R-phenyl)-6,7-dihydro-thieno[2,3-b]pyridin-6-one:

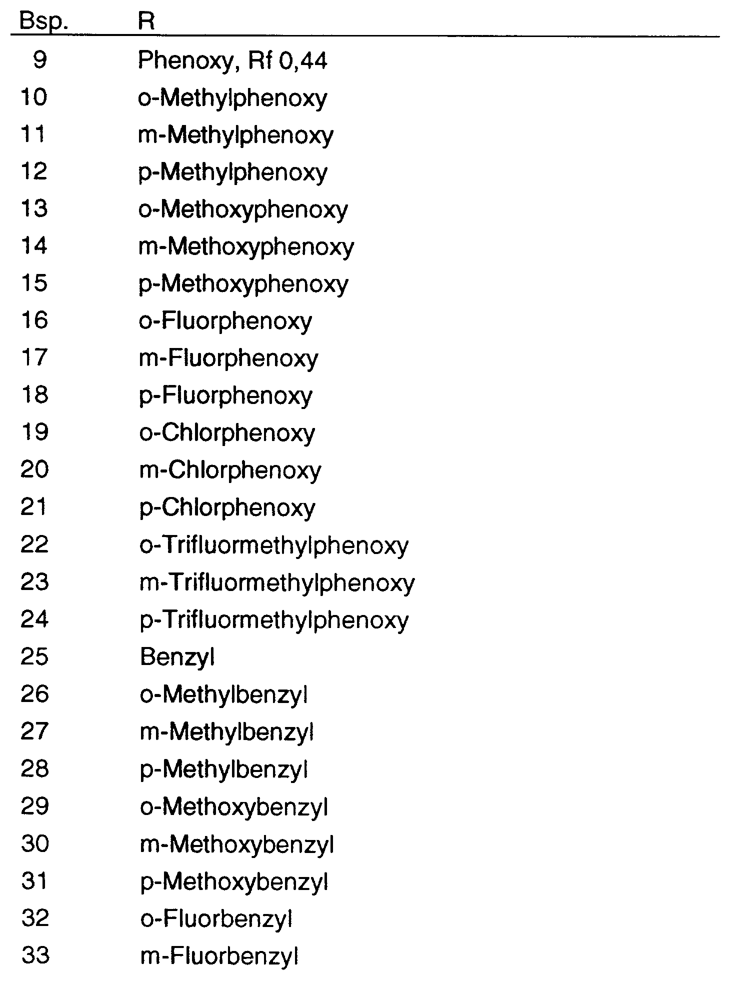

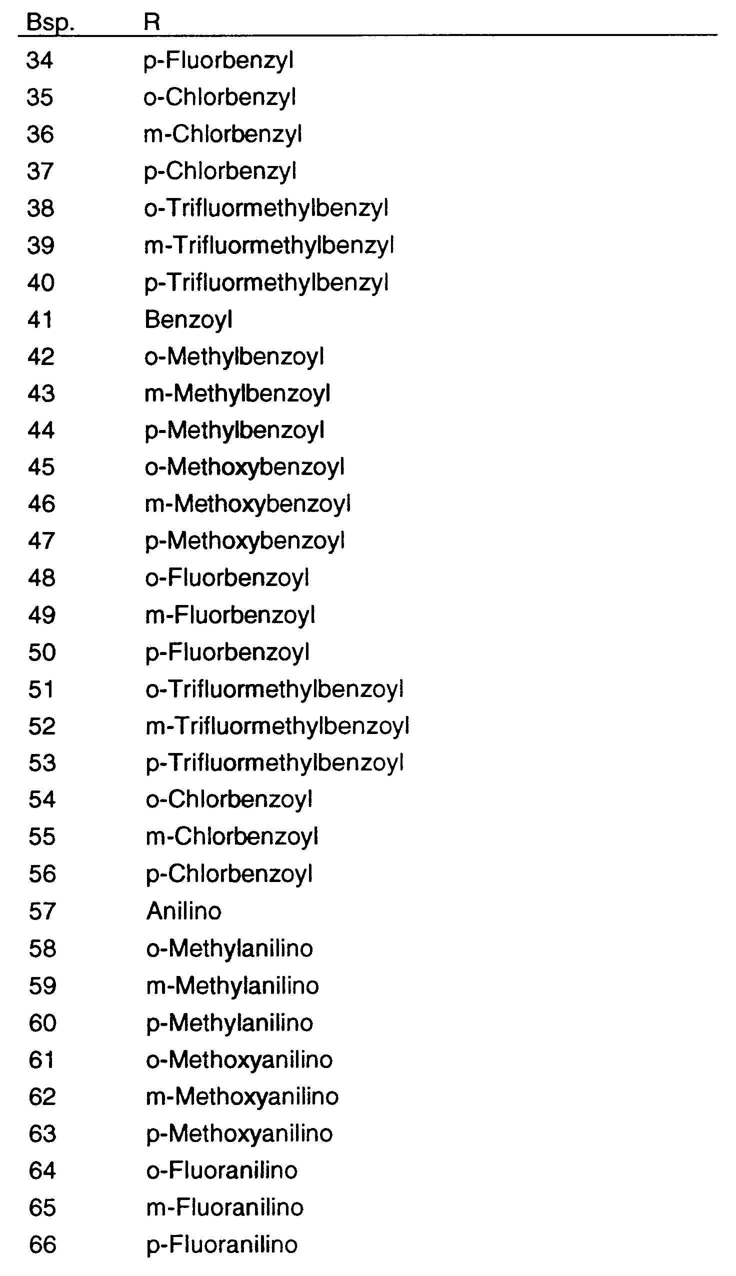

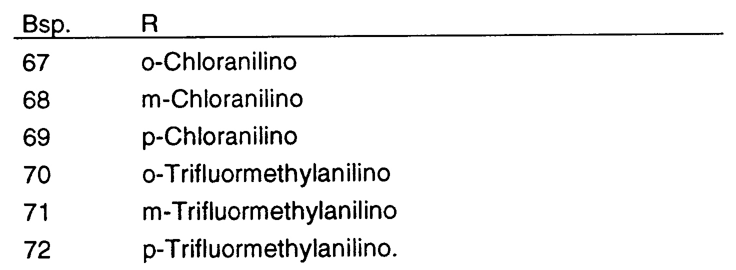

### Beispiele 73 bis 136

Analog Beispiel 1 erhält man aus den nachstehenden 2-(4-R-2-pyridyl)acetamido-4-methyl-5-chlor-thiophen-3-carbonsäure-methylestern:

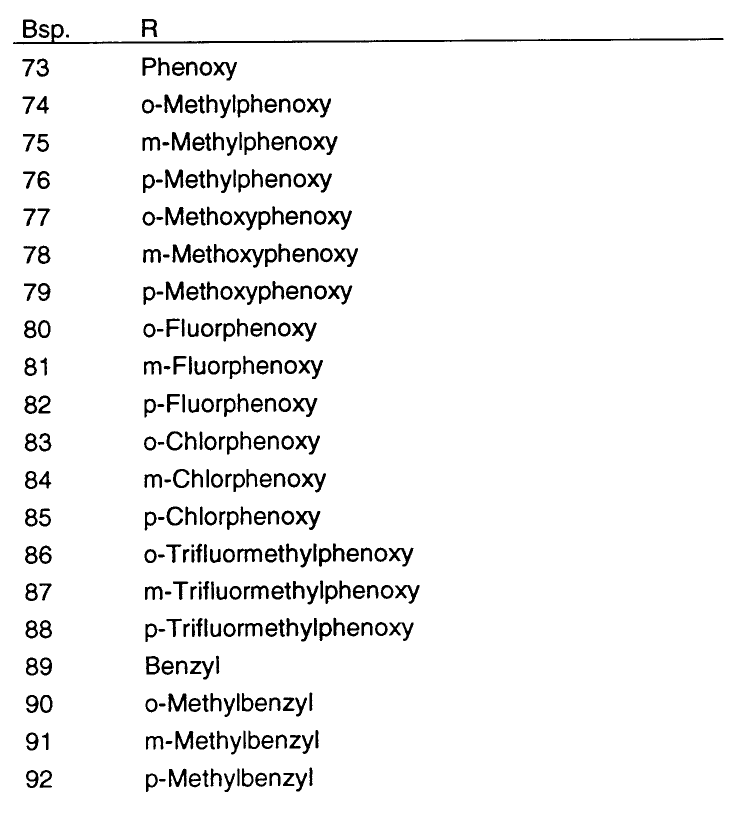

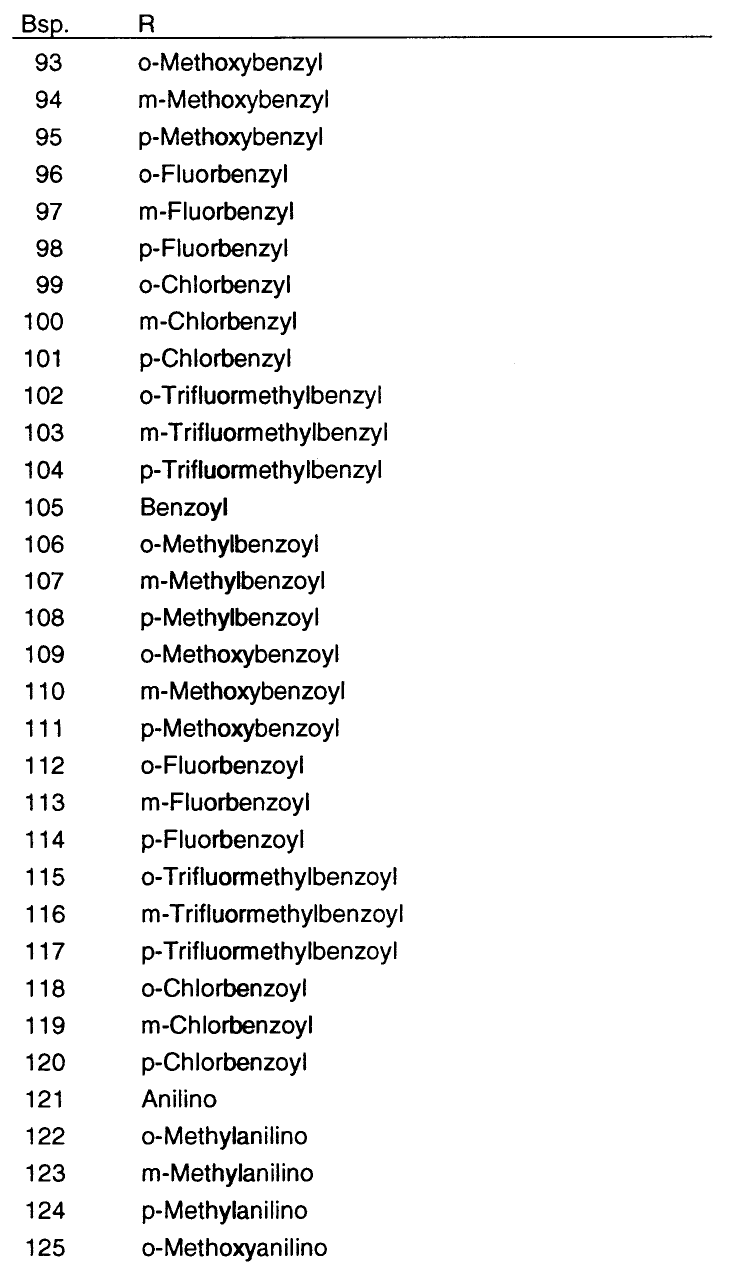

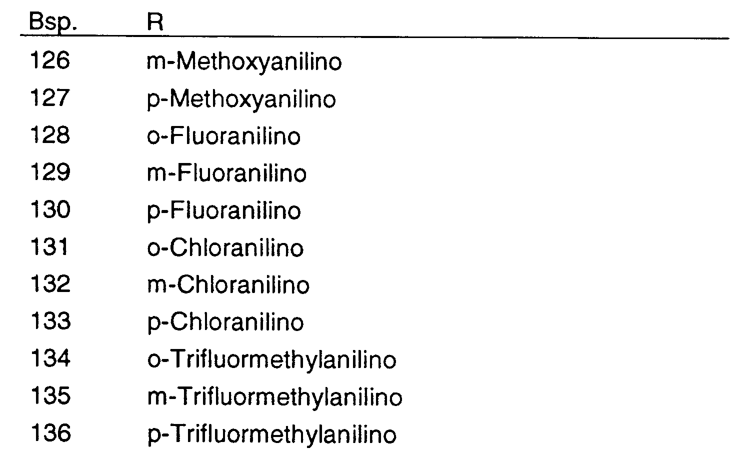

die nachstehenden 2-Chlor-3-methyl-4-hydroxy-5-(4-R-2-pyridyl)-6,7-dihydro-thieno[2,3,-b]pyridin-6-one:

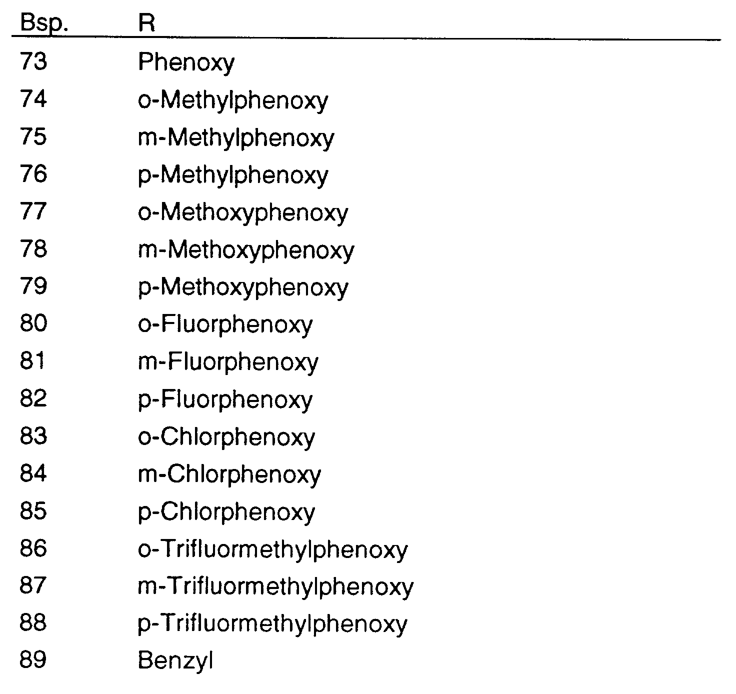

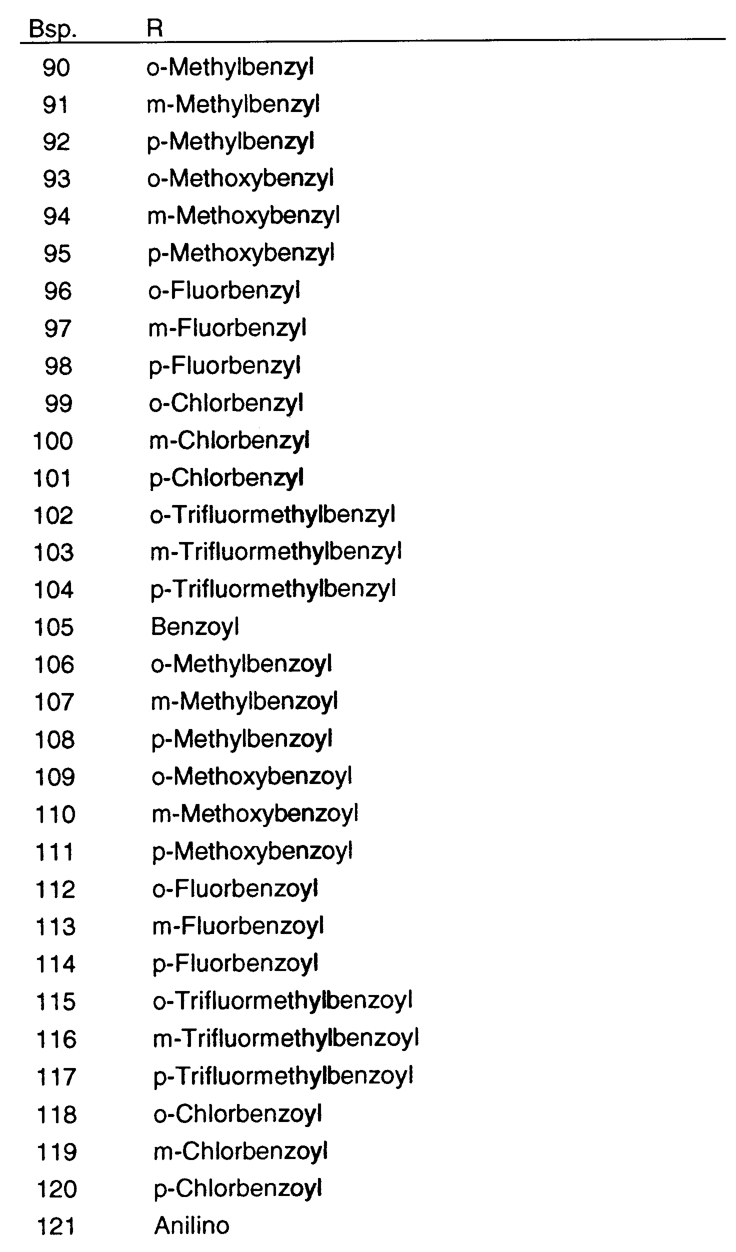

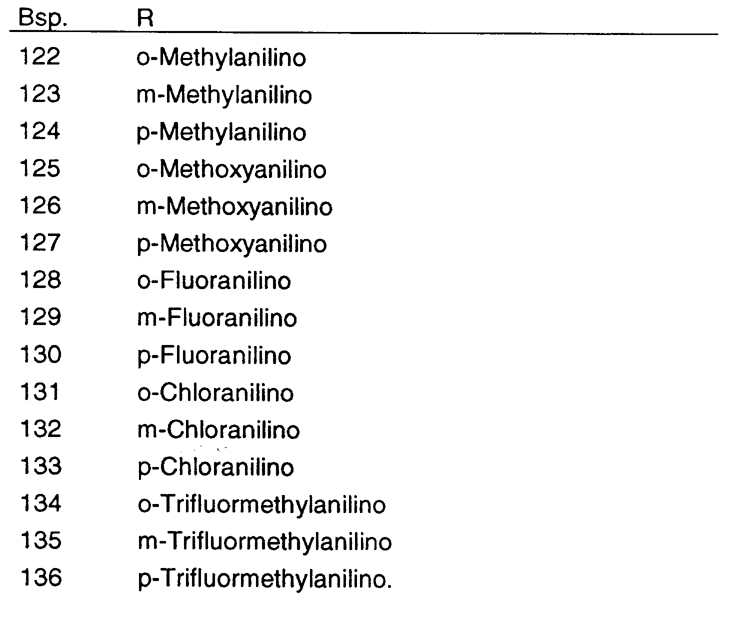

### Beispiele 137 bis 143

Analog Beispiel 1 erhält man aus den nachstehenden 2-(2-Pyridyl)-acetamido-4-R¹-5-R²-thiophen-3-carbonsäure-methylestern:

| Bsp. | R¹ | R² |
|---|---|---|
| 137 | Me | H |
| 138 | H | Me |
| 139 | Me | Me |
| 140 | H | Et |
| 141 | H | H |
| 142 | Me | Br |
| 143 | H | Br |

die nachstehenden 2-R²-3-R¹-4-hydroxy-5-(2-pyridyl)-6,7-dihydrothieno[2,3-b]pyridin-6-one:

| Bsp. | R¹ | R² |
|---|---|---|
| 137 | Me | H |
| 138 | H | Me |
| 139 | Me | Me |
| 140 | H | Et |
| 141 | H | H |
| 142 | Me | Br |
| 143 | H | Br. |

### Beispiele 143-146

Analog Beispiel 1 erhält man aus den nachstehenden 2-(3-R-Phenyl)-acetamido-4-methyl-5-chlor-thiophen-3-carbonsäure-methylestern:

| Bsp. | R |
|---|---|
| 143 | 2-Thienyl-carbonyl |
| 144 | 2-Thienyl-methyl |
| 145 | 3-Thienyl-carbonyl |
| 146 | 3-Thienyl-methyl |

die nachstehenden 2-Chlor-3-methyl-4-hydroxy-5-(3-R-phenyl)-6,7-dihydro-thieno[2,3-b]pyridin-6-one:

| Bsp. | R |
|---|---|
| 143 | 2-Thienyl-carbonyl |
| 144 | 2-Thienyl-methyl |
| 145 | 3-Thienyl-carbonyl |
| 146 | 3-Thienyl-methyl. |

### Beispiele 147-182

Analog erhält man aus den nachstehenden 2-(3-R-Phenyl)-acetamido-4-methyl-5-chlor-thiophen-3-carbonsäure-methylestern:

| Bsp. | R |
|---|---|
| 147 | o-Nitrophenoxy |
| 148 | m-Nitrophenoxy |
| 149 | p-Nitrophenoxy |
| 150 | o-N,N-Dimethylaminophenoxy |
| 151 | m-N,N-Dimethylaminophenoxy |
| 152 | p-N,N-Dimethylaminophenoxy |
| 153 | o-Acetamidophenoxy |
| 154 | m-Acetamidophenoxy |
| 155 | p-Acetamidophenoxy |
| 156 | o-Nitrobenzyl |
| 157 | m-Nitrobenzyl |
| 158 | p-Nitrobenzyl |
| 159 | o-N,N-Dimethylaminobenzyl |
| 160 | m-N,N-Dimethylaminobenzyl |
| 161 | p-N,N-Dimethylaminobenzyl |
| 162 | o-Acetamidobenzyl |
| 163 | m-Acetamidobenzyl |
| 164 | p-Acetamidobenzyl |
| 165 | o-Nitrobenzoyl |
| 166 | m-Nitrobenzoyl |
| 167 | p-Nitrobenzoyl |
| 168 | o-N,N-Dimethylaminobenzoyl |
| 169 | m-N,N-Dimethylaminobenzoyl |
| 170 | p-N,N-Dimethylaminobenzoyl |
| 171 | o-Acetamidobenzoyl |
| 172 | m-Acetamidobenzoyl |
| 173 | p-Acetamidobenzoyl |
| 174 | o-Nitroanilino |
| 175 | m-Nitroanilino |
| 176 | p-Nitroanilino |
| 177 | o-N,N-Dimethylaminoanilino |
| 178 | m-N,N-Dimethylaminoanilino |
| 179 | p-N,N-Dimethylaminoanilino |
| 180 | o-Acetamidoanilino |
| 181 | m-Acetamidoanilino |
| 182 | p-Acetamidoanilino |

die nachstehenden 2-Chlor-3-methyl-4-hydroxy-5-(3-R-phenyl)-6,7-dihydro-thieno[2,3-b]pyridin-6-one:

| Bsp. | R |
|---|---|
| 147 | o-Nitrophenoxy |
| 148 | m-Nitrophenoxy |
| 149 | p-Nitrophenoxy |
| 150 | o-N,N-Dimethylaminophenoxy |
| 151 | m-N,N-Dimethylaminophenoxy |
| 152 | p-N,N-Dimethylaminophenoxy |
| 153 | o-Acetamidophenoxy |
| 154 | m-Acetamidophenoxy |
| 155 | p-Acetamidophenoxy |
| 156 | o-Nitrobenzyl |
| 157 | m-Nitrobenzyl |
| 158 | p-Nitrobenzyl |
| 159 | o-N,N-Dimethylaminobenzyl |
| 160 | m-N,N-Dimethylaminobenzyl |
| 161 | p-N,N-Dimethylaminobenzyl |
| 162 | o-Acetamidobenzyl |
| 163 | m-Acetamidobenzyl |
| 164 | p-Acetamidobenzyl |
| 165 | o-Nitrobenzoyl |
| 166 | m-Nitrobenzoyl |
| 167 | p-Nitrobenzoyl |
| 168 | o-N,N-Dimethylaminobenzoyl |
| 169 | m-N,N-Dimethylaminobenzoyl |
| 170 | p-N,N-Dimethylaminobenzoyl |
| 171 | o-Acetamidobenzoyl |
| 172 | m-Acetamidobenzoyl |
| 173 | p-Acetamidobenzoyl |
| 174 | o-Nitroanilino |
| 175 | m-Nitroanilino |
| 176 | p-Nitroanilino |
| 177 | o-N,N-Dimethylaminoanilino |
| 178 | m-N,N-Dimethylaminoanilino |
| 179 | p-N,N-Dimethylaminoanilino |
| 180 | o-Acetamidoanilino |
| 181 | m-Acetamidoanilino |
| 182 | p-Acetamidoanilino. |

### Beispiel 183

Analog Beispiel 1 erhält man aus 3-Phenylacetamido-thiophen-2-carbonsäure-methylester durch Cyclisierung das 7-Hydroxy-6-Phenyl-4,5-dihydro-thieno[3,2-b]pyridin-5-on, R_{f} 0,31 (Dichlormethan/Methanol 10:1).

### Beispiel 184

Analog Beispiel 1 erhält man aus 4-Phenylacetamido-thiophen-3-carbonsäure-methylester die Verbindung 4-Hydroxy-3-phenyl-1,2-dihydrothieno[3,4-b]pyridin-2-on, R_{f} 0,37 (Dichlormethan/Methanol 10:1).

### Beispiel 185

Eine Lösung von 4,28 g 2-Chlor-3-methyl-4-hydroxy-5-(3-m-nitrophenoxyphenyl)-6,7-dihydrothieno[2,3-b]pyridin-6-on in 50 ml 95%igem Ethanol wird mit 20 mg Platinoxid versetzt. Man leitet so lange Wasserstoff durch die Lösung, bis drei Moläquivalente absorbiert sind. Das Platin wird abfiltriert und der Alkohol abdestilliert. Man erhält 2-Chlor-3-methyl-4-hydroxy-5-(3-m-aminophenoxy-phenyl)-6,7-dihydro-thieno[2,3-b]pyridin-6-on.

### Beispiel 186

Eine Lösung von 4,4 g 2-Chlor-3-methyl-4-hydroxy-5-(3-o-acetamidophenoxy-phenyl)-6,7-dihydro-thieno[2,3-b]pyridin-6-on in 80 ml 10%iger methanolischer KOH-Lösung wird 48 Stunden gekocht. Nach üblicher Aufarbeitung erhält man 2-Chlor-3-methyl-4-hydroxy-5-(3-o-aminophenoxy-phenyl)-6,7-dihydro-thieno[2,3-b]pyridin-6-on.

### Beispiel 187

Ein Gemisch von 4,25 g 2-Chlor-3-methyl-4-hydroxy-5-(3-p-methoxybenzoyl-phenyl)-6,7-dihydro-thieno[2,3-b]pyridin-6-on und 3,5 g Pyridinhydrochlorid wird 3 Stunden bei 160° gerührt. Nach üblicher Aufarbeitung erhält man 2-Chlor-3-methyl-4-hydroxy-5-(3-p-hydroxybenzoyl-phenyl)-6,7-dihydro-thieno[2,3-b]pyridin-6-on.

### Beispiele 188 bis 198

Analog Beispiel 1 erhält man aus den nachstehenden 2-(3-R-4-R⁶-Phenyl)-acetamido-4-R¹-5-R²-thiophen-3-carbonsäuremethylestern

| Bsp. | R | R⁶ | R¹ | R² | F. |
|---|---|---|---|---|---|
| 188 | H | F | H | H | 102,5-104° |
| 189 | H | H | Ethyl | H | 116-117° |
| 190 | H | H | Ethyl | Br | 92-93° |
| 191 | H | H | Ethyl | Cl | 89,5-91,5° |
| 192 | OC₆H₅ | H | Methyl | H | 83-85° |
| 193 | OC₆H₅ | H | Ethyl | Cl | 116-118° |
| 194 | H | H | CH(CH₃)₂ | Cl | 70-71° |
| 195 | OC₆H₅ | H | CH(CH₃)₂ | Cl | 61-63° |
| 196 | OC₆H₅ | H | cyclohexyl | Cl | 83,5-84,5° |
| 197 | OC₆H₅ | H | cyclopropyl | Cl | 95-97° |
| 198 | OC₆H₅ | H | tert.-butyl | Cl | 63,5-66° |

die nachstehenden 2-R²-3-R¹-4-hydroxy-5-(3-R-4-R⁶-phenyl)-6,7-dihydrothieno[2,3-b]-pyridin-6-one:

| Bsp. | R | R⁶ | R¹ | R² | Rf (CH₂Cl₂/MeOH 10:1) |
|---|---|---|---|---|---|
| 188 | H | F | H | H | 0,34 |
| 189 | H | H | Ethyl | H | 0,38 |
| 190 | H | H | Ethyl | Br | 0,35 |
| 191 | H | H | Ethyl | Cl | 0,4 |
| 192 | OC₆H₅ | H | Methyl | H | 0,39 |
| 193 | OC₆H₅ | H | Ethyl | Cl | 0,59 |
| 194 | H | H | CH(CH₃)₂ | Cl | 0,56 |
| 195 | OC₆H₅ | H | CH(CH₃)₂ | Cl | 0,58 |
| 196 | OC₆H₅ | H | cyclohexyl | Cl | 0,44 |
| 197 | OC₆H₅ | H | cyclopropyl | Cl | 0,42 |
| 198 | OC₆H₅ | H | tert.-butyl | Cl | 0,45 |

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

### Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

1. Thienopyridone der Formel I 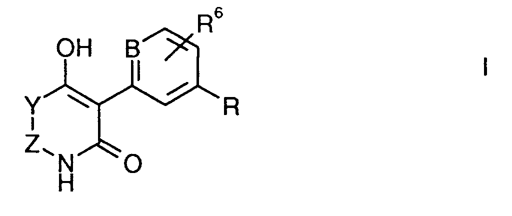 worin
B CH oder N, 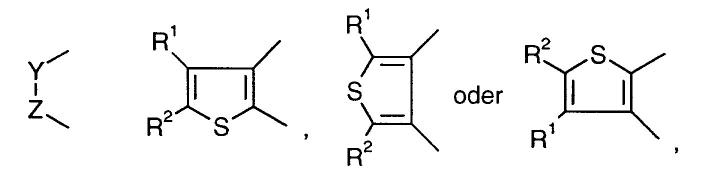
R H, 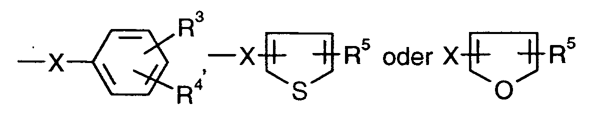
R¹, R² und R⁵ jeweils H, A oder Hal,
R³ und R⁴ jeweils H, A, OH, OA, Hal, CF₃, NO₂, NH₂, NHA, N(A)₂ oder NHAc,
R⁶ H oder Hal,
X -CH₂-, -CO-, -O-, -NH-, -NA- oder S,
A Alkyl mit 1-4 C-Atomen,
Ac Alkanoyl mit 1-6 C-Atomen oder Benzoyl und
Hal F, Cl, Br oder I
bedeuten,
sowie ihre Salze.

2. 2-Chlor-3-methyl-4-hydroxy-5-phenyl-6,7-dihydrothieno[2,3-b]pyridin-6-on.

3. Verfahren zur Herstellung eines Thienopyridons der Formel I nach Anspruch 1, dadurch gekennzeichnet, daß man eine Verbindung der Formel II 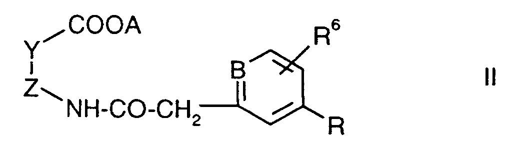 worin A, B, R, R⁶ und -Y-Z- die in Anspruch 1 angegebenen Bedeutungen haben,
mit einem Cyclisierungsmittel behandelt,
und daß man gegebenenfalls in einer Verbindung der Formel I einen Rest R in einen anderen Rest R umwandelt und/oder eine Verbindung der Formel I durch Behandeln mit einer Säure oder Base in eines ihrer Salze überführt.

4. Verfahren zur Herstellung einer pharmazeutischen Zubereitung, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verwendung einer Verbindung der Formel I nach Anspruch 1 oder eines ihrer physiologisch unbedenklichen Salze zur Herstellung eines Arzneimittels.

7. Verwendung einer Verbindung der Formel I nach Anspruch 1 oder eines ihrer physiologisch unbedenklichen Salze bei der Bekämpfung von Krankheiten, insbesondere cerebraler Ischämie und Schizophrenie.
